# EUROPEAN PATENT SPECIFICATION

(11) **EP 3 991 689 B1**
(45) Date of publication and mention of the grant of the patent: **31.07.2024**
(21) Application number: 21203125.6
(22) Date of filing: 18.10.2021
(51) Int. Cl.: A61C 17/02, A61B 17/24

(54) **ORAL HYGIENE DEVICE MEMBER**
MUNDHYGIENEVORRICHTUNGSELEMENT
ÉLÉMENT DE DISPOSITIF D'HYGIÈNE BUCCALE

(30) Priority: 27.10.2020 JP 2020179778
(43) Date of publication of application: 04.05.2022
(73) Proprietor: Panasonic Intellectual Property Management Co., Ltd., Osaka-shi, Osaka 540-6207 (JP)
(72) Inventor: Honda, Misa, Osaka-shi, Osaka (JP); Taniguchi, Shinichi, Osaka-shi, Osaka (JP); Inoue, Hiroki, Osaka-shi, Osaka (JP)
(74) Representative: Grünecker Patent- und Rechtsanwälte PartG mbB

(56) References cited:
- WO-A1-2004/021958
- CN-Y- 2 668 058
- DE-A1- 19 959 188
- FR-A- 468 322
- US-A1- 2019 040 561

## Description

### BACKGROUND

### 1. Technical Field

The present disclosure relates to an oral hygiene device member.

### 2. Description of the Related Art

CN 2 668 058 Y discloses an oral hygiene device member having the features of the preamble of claim 1.

As disclosed in the following PTL 1, as an oral hygiene device member, a technique is known in which a bristle part is provided on a sheet-shaped base material and a site to be cleaned such as a tongue or an oral mucosa is scrubbed with the bristle part.

In PTL 1, the bristle part comprises a first filament and a second filament that protrude from the sheet-shaped base material. The first filament has a loop shape in which both ends have been fixed to the sheet-shaped base material, and the second filament has one end fixed to the sheet-shaped base material and another end being a free end. This can provide an excellent feeling of touch and makes it possible to satisfactorily clean an entire oral cavity.

### Citation List

### Patent Literature

PTL 1: Unexamined Japanese Patent Publication No. 2013-198672

### SUMMARY

However, in the related art described above, while it is possible to provide an excellent feeling of touch and makes it possible to satisfactorily clean an entire oral cavity, the first filament and the second filament are densely provided on the sheet-shaped base material. Therefore, there is a problem that the sheet-shaped base material is immediately fouled when the site to be cleaned is scrubbed.

According to the related art described above, the first filament and the second filament are brought into direct contact with a site to be cleaned such as a tongue or an oral mucosa when the site to be cleaned is scrubbed. Therefore, there is a possibility that a burden applied to the site to be cleaned becomes large.

Therefore, an object of the present disclosure is to provide an oral hygiene device member capable of further reducing a burden on a target object in an oral cavity and capable of maintaining a cleaner state while suppressing deterioration in cleaning performance.

To achieve this object, an oral hygiene device member according to claim 1 is provided. An oral hygiene device member according to one aspect disclosed herein: a handle having a flow path through which liquid is allowed to pass; and a head comprising a head body connected to one end side of the handle, and a head material held by the head body. The head material comprises a cleaning part. The cleaning part is exposed from the head body in a state of being held by the head body. The cleaning part is formed of fibers that clean a target object in an oral cavity. A liquid supply path is formed in the head. The liquid supply path communicates with the flow path and supplies liquid to the cleaning part.

According to the present disclosure, it is possible to provide an oral hygiene device member capable of further reducing a burden on a target object in an oral cavity and capable of maintaining a cleaner state while suppressing deterioration in cleaning performance.

### BRIEF DESCRIPTION OF THE DRAWINGS

FIG. 1 is a perspective view illustrating one example of an oral hygiene device according to an exemplary embodiment;
FIG. 2 is a side view illustrating an oral hygiene device member according to the exemplary embodiment;
FIG. 3 is an exploded perspective view illustrating the oral hygiene device member according to the exemplary embodiment;
FIG. 4 is a side view illustrating a handle and a tubular part according to the exemplary embodiment;
FIG. 5 is a front view illustrating the handle and the tubular part according to the exemplary embodiment;
FIG. 6 is a rear view illustrating the handle and the tubular part according to the exemplary embodiment;
FIG. 7 is a partially enlarged side cross-sectional view illustrating the handle and the tubular part according to the exemplary embodiment;
FIG. 8 is a partially enlarged perspective view illustrating the handle and the tubular part according to the exemplary embodiment;
FIG. 9 is a plan view illustrating a head material according to the exemplary embodiment;
FIG. 10 is a cross-sectional view illustrating the head material according to the exemplary embodiment;
FIG. 11 is a view for explaining a cleaning part of the head material according to the exemplary embodiment;
FIG. 12 is a cross-sectional view illustrating a fiber used in the head material according to the exemplary embodiment;
FIG. 13 is a plan view illustrating a sheet-shaped member usable for forming the head material according to the exemplary embodiment;
FIG. 14 is a perspective view of a holder according to the exemplary embodiment as viewed from one direction;
FIG. 15 is a perspective view of the holder according to the exemplary embodiment as viewed from another direction;
FIG. 16 is a perspective view illustrating a cover according to the exemplary embodiment;
FIG. 17 is a side cross-sectional view illustrating a head according to the exemplary embodiment;
FIG. 18A is a view for explaining one example of a retainer and an outlet formed in a head body according to the exemplary embodiment;
FIG. 18B is an enlarged view illustrating a part of the retainer and the outlet in FIG. 18A;
FIG. 19 is a view for explaining a first modification of the retainer and the outlet formed in the head body according to the exemplary embodiment;
FIG. 20 is a view for explaining a second modification of the retainer and the outlet formed in the head body according to the exemplary embodiment;
FIG. 21 is a view for explaining a third modification of the retainer and the outlet formed in the head body according to the exemplary embodiment; and
FIG. 22 is a view for explaining a fourth modification of the retainer and the outlet formed in the head body according to the exemplary embodiment.

### DETAILED DESCRIPTION

Hereinafter, an exemplary embodiment will be described in detail with reference to the drawings. It is noted that a more detailed description than need may be omitted. For example, the detailed description of already well-known matters and the overlap description of substantially the same configurations may be omitted.

Note that the attached drawings and the following description are provided for those skilled in the art to fully understand the present disclosure, and are not intended to limit the subject matter as described in the appended claims.

Hereinafter, as the oral hygiene device member, an oral hygiene device nozzle usable as a part of various attachments detachably attached to a device body of an oral hygiene device will be exemplified.

Further, in the following exemplary embodiment, a description will be made with directions of the oral hygiene device nozzle in which a direction along a longitudinal direction of a nozzle handle (hereinafter, which may be referred to simply as handle) is defined as up-down direction Z, and a direction along an axial direction of a tubular part is defined as front-back direction X. A description will be made while a direction orthogonal to up-down direction Z and front-back direction X is defined as width direction Y.

Further, in the following exemplary embodiment, a description will be made while a head side in a state where the head is positioned on an upper side is defined as an upper side in up-down direction Z, and a side where a cleaning part of a head material is exposed (namely, a side where the head material protrudes from the head body) is defined as a front side in front-back direction X.

### (Exemplary embodiment)

Hereinafter, an example of an oral hygiene device and an oral hygiene device nozzle according to an exemplary embodiment will be described.

### [One example of configuration of oral hygiene device]

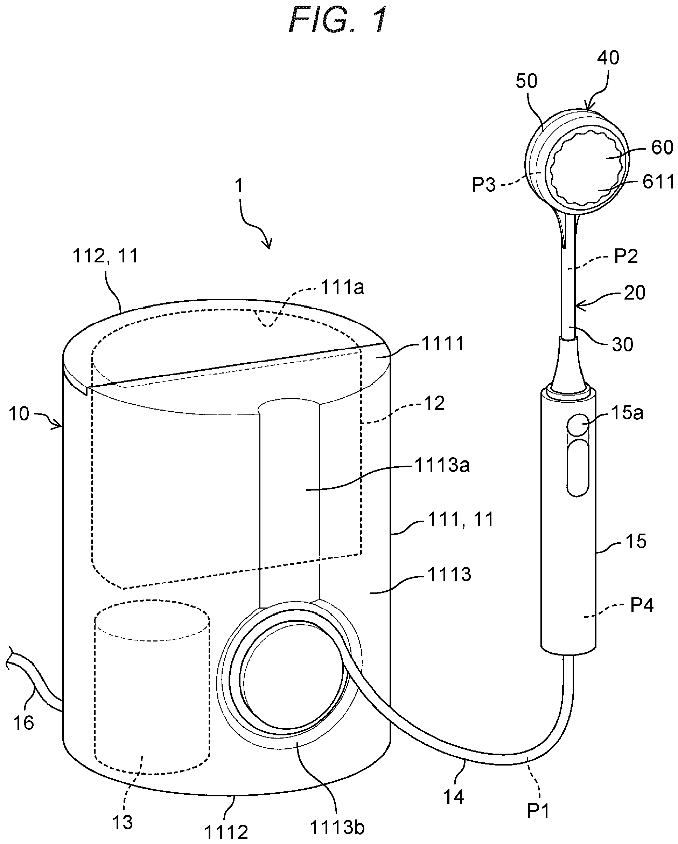

First, one example of a configuration of an oral hygiene device will be described with reference to FIG. 1.

As illustrated in FIG. 1, oral hygiene device 1 according to the present exemplary embodiment comprises device body 10 and oral hygiene device nozzle (i.e., oral hygiene device member) 20 detachably attached to device body 10.

Here, in the present exemplary embodiment, oral hygiene device nozzle 20 comprises nozzle handle (i.e., handle) 30 elongated in one direction, and head 40 connected to one end side of nozzle handle 30. Head 40 comprises: head body 50; and head material 60 having fibrous cleaning part 611 and held by head body 50. This oral hygiene device nozzle 20 is configured to allows liquid to be supplied to fibrous cleaning part 611.

Specifically, flow path P2 through which liquid can pass is formed in nozzle handle 30 of oral hygiene device nozzle 20, and liquid supply path P3 that communicates with flow path P2 and can supply liquid to cleaning part 611 is formed in head 40. On the other hand, as will be described later, device body 10 comprises hose 14 in which introduction path P1 through which liquid can be introduced into flow path P2 of oral hygiene device nozzle 20 has been formed.

As described above, oral hygiene device 1 formed by attaching oral hygiene device nozzle 20 according to the present exemplary embodiment to device body 10 is a device that can clean an inside of an oral cavity with cleaning part 611 while liquid is supplied to cleaning part 611, in a state where head material 60 has been inserted into the oral cavity of a person who is a cleaning target, such as a user. That is, oral hygiene device 1 according to the present exemplary embodiment can clean the inside of the oral cavity with liquid together with a brushing effect by fibrous cleaning part 611, and has a function as an oral cavity washing device.

As the liquid supplied through oral hygiene device nozzle 20, water can be used, but it is possible to use various kinds of liquid without limiting to water. For example, washing liquid in which a cleaning agent has been mixed in water can be used.

According to the present exemplary embodiment, device body 10 comprises liquid storage tank (i.e., liquid storage) 12 capable of storing liquid, and pump 13 for introducing the liquid in liquid storage tank 12 into flow path P2 of oral hygiene device nozzle 20. Further, device body 10 comprises hose 14 connected to liquid storage tank 12, and grip 15 having one end connected to hose 14 and another end attachable with oral hygiene device nozzle 20.

Liquid storage tank 12 stores liquid to be supplied to oral hygiene device nozzle 20. Note that device body 10 does not need to have liquid storage tank 12, and for example, liquid such as tap water can also be directly drawn from the outside.

Pump 13 draws in the liquid stored in liquid storage tank 12 and sends out the liquid to grip 15 through hose 14. As pump 13, for example, a pump of a piston pump type can be used. However, a method for pump 13 is not particularly limited.

Grip 15 is a member that can be held by a user or the like when oral hygiene device 1 is used, and has a substantially cylindrical shape. To one distal end in an axial direction of grip 15, oral hygiene device nozzle 20 is detachably attached. Then, to another end of grip 15 in the axial direction, hose 14 is connected.

Inside of this hose 14, introduction path P1 having one end communicating with liquid storage tank 12 is formed. Introduction path P1 communicates with flow path P2 of nozzle handle 30 via introduction path P1 formed inside grip 15. Inside of grip 15, flow path P4 is provided. Flow path P4 communicates with introduction path P1 and flow path P2. Therefore, the liquid in liquid storage tank 12 is introduced into grip 15 via hose 14, and the liquid introduced into grip 15 is guided to flow path P2 and liquid supply path P3 in oral hygiene device nozzle 20.

Further, in the present exemplary embodiment, grip 15 comprises operation switch 15a partially exposed to a front surface side. When the user or the like operates operation switch 15a while holding grip 15, it is possible to operate starting and ending of liquid ejection.

In addition, device body 10 may comprise case 11 that accommodates liquid storage tank 12 and pump 13. In the present exemplary embodiment, device body 10 that comprises case 11 having a substantially cylindrical outer shape is exemplified.

This case 11 comprises case body 111 that accommodates liquid storage tank 12 and pump 13, and upper lid 112 turnably attached to case body 111 and openably/closably covering opening 111a opened upward of case body 111.

In the present exemplary embodiment, case body 111 comprises: top wall 1111 that is a substantially semicircular plate member; bottom wall 1112 that is a substantially circular plate member; and peripheral wall 1113 that is connected so as to rise from a peripheral edge of bottom wall 1112 and has a part of an upper end connected to an arc portion of top wall 1111.

Therefore, semicircular opening 111a is to be formed to open upward in a portion of case body 111 not covered with top wall 1111.

Further, liquid storage tank 12 is accommodated in case body 111 in a state where an opening of liquid storage tank 12 communicates with opening 111a of case body 111, and upper lid 112 is attached to case body 111 so as to openably close this opening 111a. Then, by the user or the like opening upper lid 112, the liquid can be replenished to liquid storage tank 12.

In addition, at least one selected from the group consisting of grip holder 1113a and hose holder 1113b may be provided on peripheral wall 1113. For example, if grip holder 1113a is provided on peripheral wall 1113, grip 15 can be engaged with and held by grip holder 1113a when oral hygiene device 1 (i.e., device body 10) is not in use. Further, if hose holder 1113b is provided on peripheral wall 1113, hose 14 can be wound around hose holder 1113b to be held when the oral hygiene device 1 (i.e., device body 10) is not in use.

Note that a control board or the like serving as a controller that controls an operation of pump 13 or the like may be installed inside case 11.

In addition, the present exemplary embodiment shows an example in which power supply for operating pump 13 and the like is supplied from the outside via power cord 16. However, the configuration of oral hygiene device 1 (i.e., device body 10) is not limited to such a configuration, and for example, it is also possible to operate pump 13 or the like in a rechargeable manner by providing a rechargeable battery in case 11.

Further, on an outer surface of case 11, an operation button or the like to adjust strength (e.g., a flow rate per unit time) of a flow of liquid supplied from oral hygiene device nozzle 20 may be installed.

In addition, case 11 and the like can be formed using a material such as a synthetic resin, but can be formed using various materials without limiting to this.

### [One example of configuration of oral hygiene device nozzle]

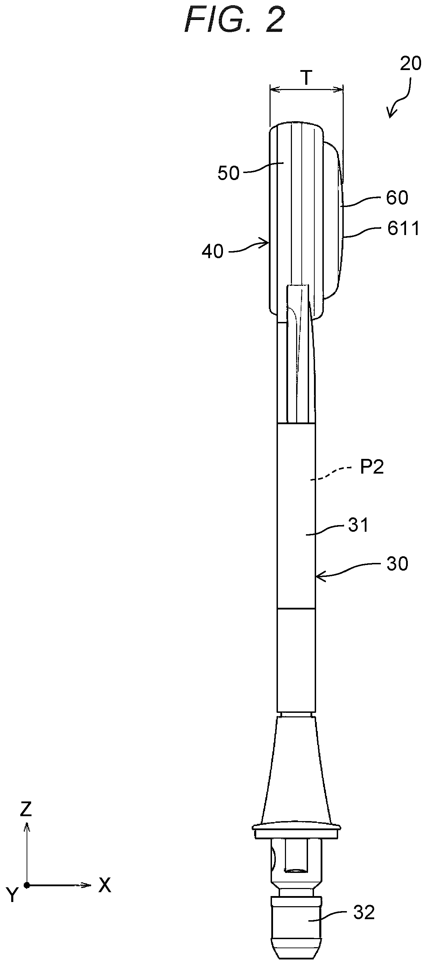

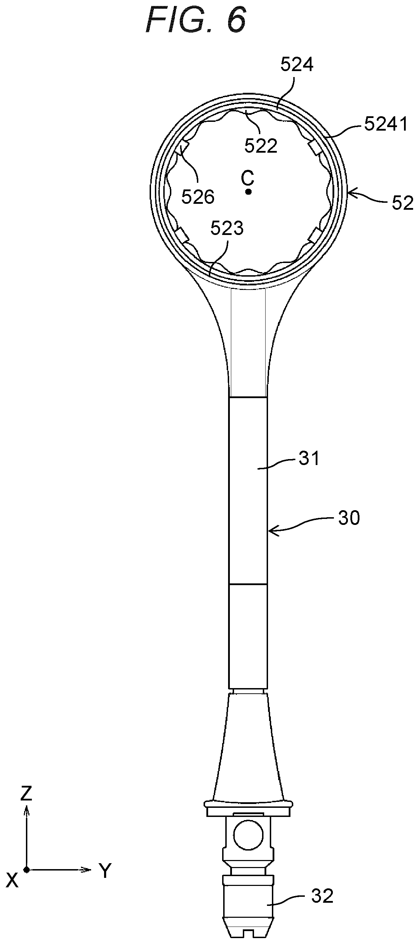

Next, one example of a configuration of an oral hygiene device nozzle will be described with reference to FIGS. 2 to 18B.

As described above, oral hygiene device nozzle 20 according to the present exemplary embodiment is detachably attached to grip 15 of device body 10 of oral hygiene device 1.

Further, as described above, oral hygiene device nozzle 20 is used as a part of various attachments detachably attached to device body 10 of oral hygiene device 1.

Examples of another attachment detachably attached to device body 10 of oral hygiene device 1 comprise, for example, a nozzle attachment capable of cleaning an oral cavity of the user or the like by ejecting pressurized liquid (e.g., water, washing water, or the like) from a nozzle at a distal end. This makes it possible to properly use a plurality of types of attachments according to applications, and makes it possible to more effectively maintain a good hygienic state in the oral cavity. Note that, instead of using oral hygiene device nozzle 20 as a part of various attachments, oral hygiene device 1 may be made to be dedicated to oral hygiene device nozzle 20.

FIG. 2 is a side view illustrating oral hygiene device nozzle 20. As illustrated in FIG. 2, oral hygiene device nozzle 20 comprises nozzle handle (i.e., handle) 30 having flow path P2 through which liquid can pass. Further, oral hygiene device nozzle 20 comprises head 40 having: head body 50 connected to one end side (see upper end in FIG. 2) of nozzle handle 30; and head material 60 held by head body 50.

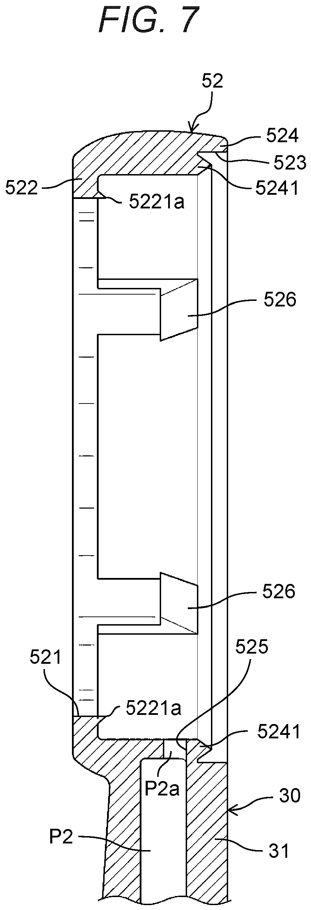

Nozzle handle 30 has a cylindrical shape elongated in up-down direction Z, and can be formed using a material such as synthetic resin, for example. However, nozzle handle 30 can also be formed using other materials such as metal. Then, inside of nozzle handle 30, flow path P2 extending in up-down direction Z is formed (see FIG. 7).

In the present exemplary embodiment, nozzle handle 30 comprises main body 31 to which head 40 is connected, and protrusion 32 that is connected below main body 31 in up-down direction Z and is engaged with one end of grip 15 in an axial direction. By protrusion 32 being inserted into and engaged with an engagement recess (not illustrated) formed in grip 15, oral hygiene device nozzle 20 is attached to grip 15.

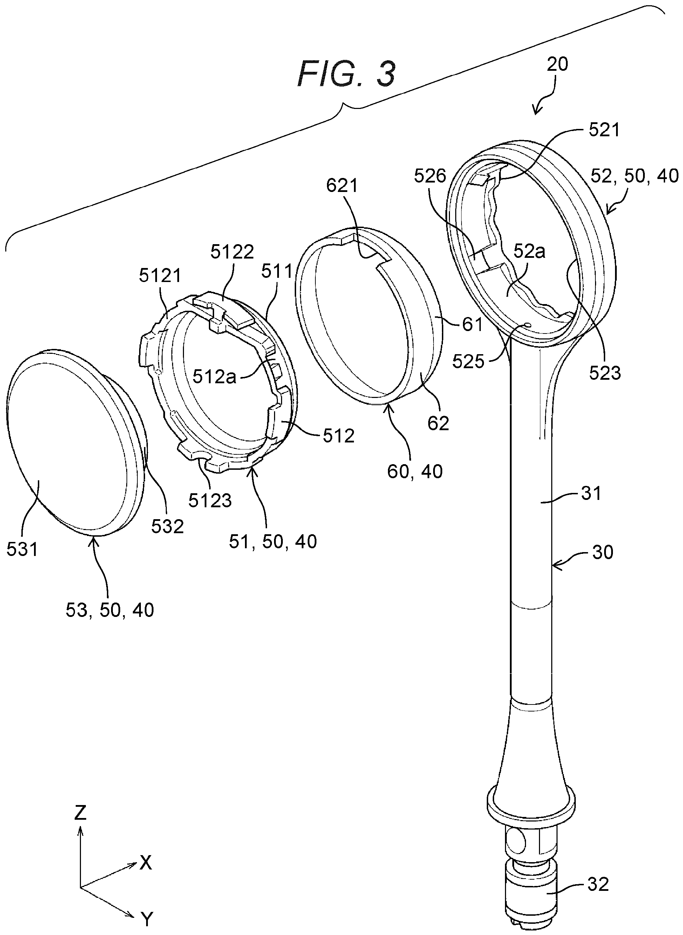

On the other hand, head 40 has a substantially disk shape, and is connected to an upper end of main body 31 in a state where an axial direction thereof is along front-back direction X. FIG. 3 is an exploded perspective view illustrating oral hygiene device nozzle 20. As illustrated in FIG. 3, head 40 comprises head body 50 connected to the upper end (i.e., one end side) of nozzle handle 30 (i.e., main body 31), and head material 60 held by head body 50.

Further, head body 50 comprises: holder (i.e., base) 51 on which head material 60 is placed; tubular part 52 that is connected to the upper end of nozzle handle 30 (i.e., main body 31) and in which holder 51 can be inserted and held; and cover 53 that closes a back side of tubular part 52 (see FIG. 3).

While holder 51, tubular part 52, cover 53, and head material 60 can also be formed using, for example, a material such as a synthetic resin, components other than head material 60 can be formed using other materials such as metal.

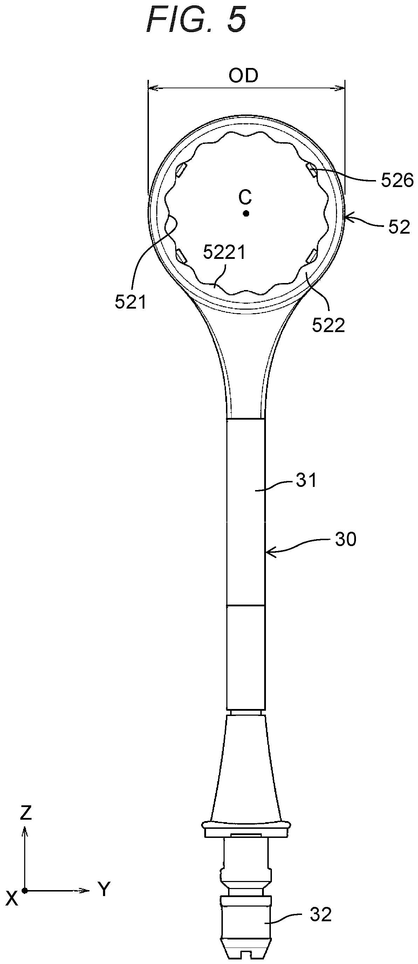

Here, in the present exemplary embodiment, head 40 is formed to have thickness T (see FIG. 2) ranging from 5 mm to 15 mm, inclusive. Further, head 40 is formed such that a maximum dimension (i.e., size in width direction Y) of outer diameter OD (see the front view illustrated in FIG. 5) ranges from 15 mm to 40 mm, inclusive. If thickness T of entire head 40 is smaller than 5 mm, or the maximum dimension of outer diameter OD of entire head 40 becomes smaller than 15 mm, it may be difficult to maintain durability during use. Further, when thickness T of entire head 40 is larger than 15 mm or the maximum dimension of outer diameter OD of entire head 40 is larger than 40 mm, a sense of discomfort may be generated when head 40 is put into the oral cavity, and a feeling of use of oral hygiene device nozzle 20 may be deteriorated.

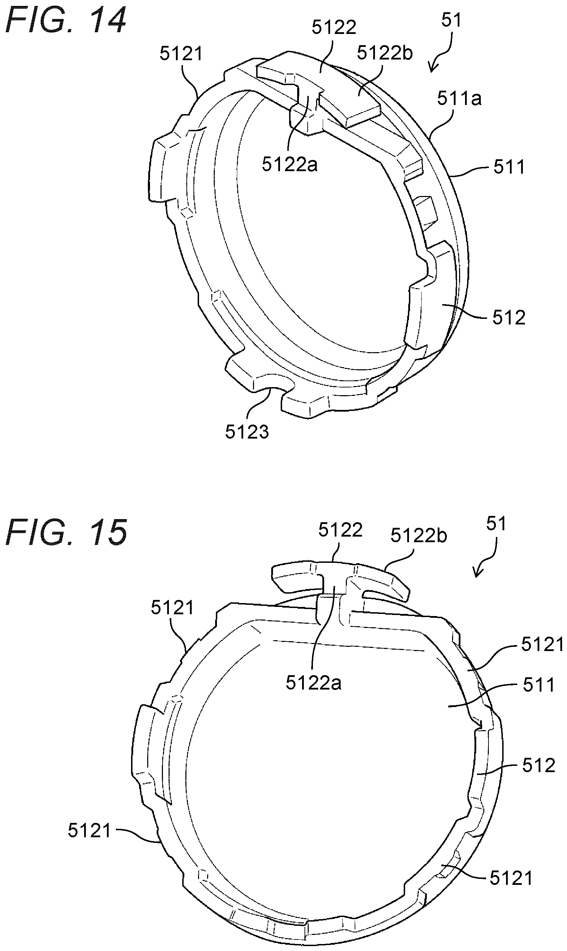

Further, as illustrated in FIG. 3, holder 51 comprises: top wall part 511 having a substantially disk shape; and tubular body 512 having a substantially cylindrical shape and extending from an outer peripheral edge of top wall part 511 toward a back side in front-back direction X (i.e., toward another side in a direction of central axis C (see FIG. 5) of tubular part 52). Top surface (i.e., front surface) 511a of top wall part 511 is a placement surface on which head material 60 is placed (see FIG. 14 that is a perspective view of holder 51).

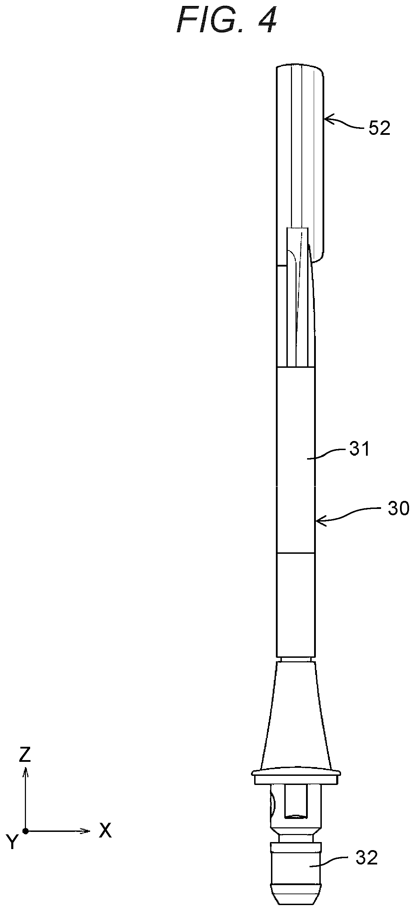

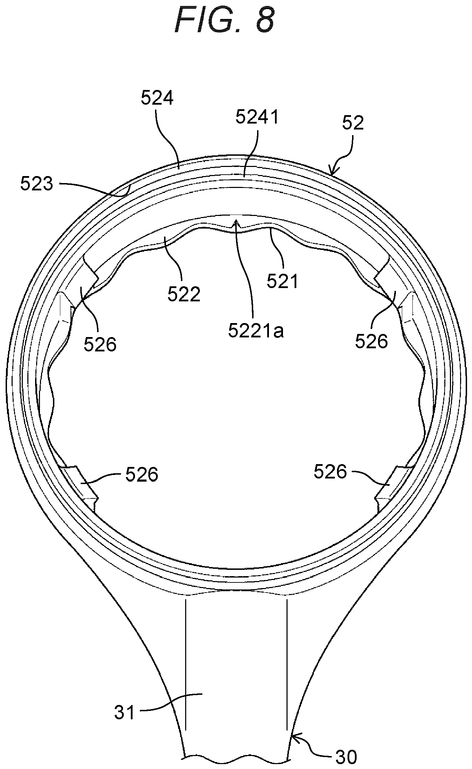

FIGS. 4, 5, and 6 are a side view, a front view, and a rear view, respectively, illustrating nozzle handle 30 (i.e., handle) and tubular part 52. FIGS. 7 and 8 are a side cross-sectional view and a perspective view, respectively, illustrating nozzle handle 30 and tubular part 52, in a partially enlarged manner. As illustrated in FIGS. 4 to 8, tubular part 52 has a substantially cylindrical shape penetrating in front-back direction X, and has first opening 521 formed on a front side in front-back direction X, and has second opening 523 formed on a back side in front-back direction X. In the present exemplary embodiment, this tubular part 52 is formed integrally with nozzle handle 30.

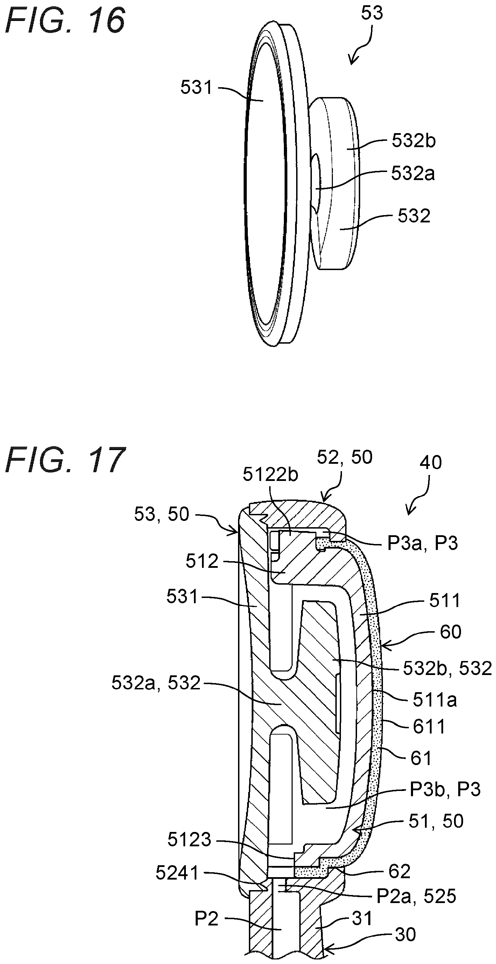

Here, in the present exemplary embodiment, tubular part 52 is formed such that an inner diameter of tubular part 52 is slightly larger than an outer diameter of tubular body 512. When holder 51 is inserted into tubular part 52, gap P3a is formed between outer peripheral surface 512a of tubular body 512 and inner peripheral surface 52a of tubular part 52 (see FIG. 17 that is a side cross-sectional view of head 40). This gap P3a constitutes a part of liquid supply path P3 formed in head 40.

In the present exemplary embodiment, at four locations on inner peripheral surface 52a of tubular part 52, locking claws 526 that lock holder (i.e., base) 51 are formed (see FIG. 8). On the other hand, at positions corresponding to locking claws 526 on outer peripheral surface 512a of tubular body 512 of holder (i.e., base) 51, portions to be locked 5121 are formed, which are to be respectively locked to locking claws 526 (see FIGS. 14 and 15 that are perspective views of holder 51).

When holder 51 is inserted into tubular part 52, portion to be locked 5121 of holder 51 is locked to locking claw 526, which inhibits backward movement of holder 51 in front-back direction X and come-off from tubular part 52. Thus, holder 51 is held by tubular part 52.

Further, in the present exemplary embodiment, peripheral edge 522 extending toward central axis C is formed around the first opening 521 of tubular part 52, and this peripheral edge 522 inhibits forward movement of holder 51 in front-back direction X and come-off from tubular part 52.

Further, cover 53 comprises: lid body 531 that closes second opening 523 located on a back side of tubular part 52 in front-back direction X (i.e., on another side in a direction of central axis C); and protrusion 532 protruding from a center of a front part of lid body 531 and inserted into tubular body 512 of holder 51 (see FIG. 3 and FIG. 16 that is a perspective view of cover 53).

When cover 53 is moved from a back side to a front side of tubular part 52 in a state where protrusion 532 protrudes forward in front-back direction X, second opening 523 is closed by lid body 531.

In the present exemplary embodiment, welding rib 5241 is formed over the entire circumference of peripheral edge 524 of tubular part 52 around the second opening 523 (see FIGS. 7 and 8). By welding rib 5241 being welded to lid body 531 through ultrasonic welding or the like, sealability of head 40 on the cover 53 side (i.e., back side) is secured. This inhibits leakage of liquid supplied to liquid supply path P3, from the cover 53 (i.e., back side).

As illustrated in FIG. 16, in the present exemplary embodiment, protrusion 532 comprises prop 532a protruding from a center of a front part of lid body 531, and disk part 532b connected to a front end of prop 532a. Disk part 532b is formed so as to expand radially outward from a front end of prop 532a, and this disk part 532b is inserted into tubular body 512 of holder 51.

In the present exemplary embodiment, inner space P3b formed inside tubular body 512 of holder 51 also constitutes a part of liquid supply path P3 (see FIG. 17). Therefore, when disk part 532b is inserted into tubular body 512 of holder 51, a volume of liquid supply path P3 is reduced by protrusion 532. Thus, the liquid does not remain in liquid supply path P3 as much as possible after use.

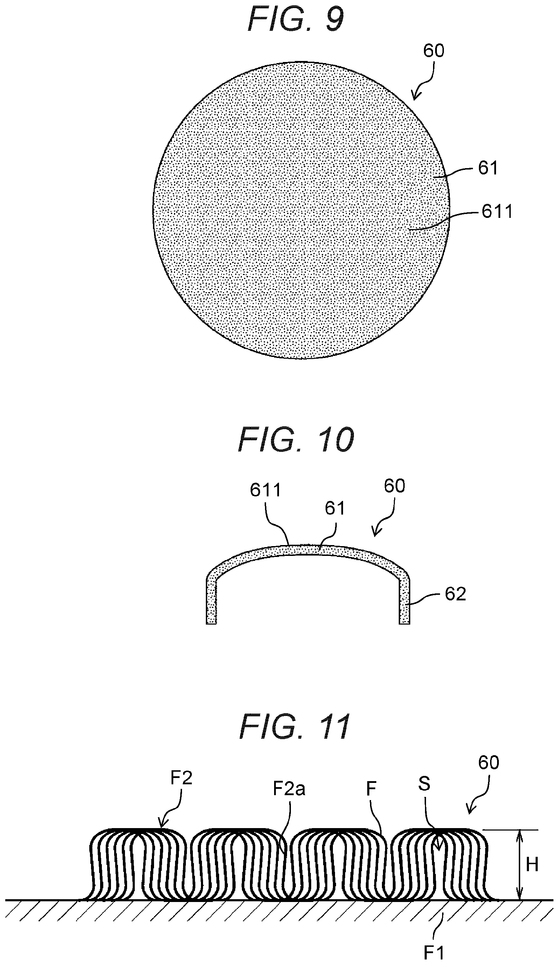

Further, head material 60 comprises fibrous cleaning part 611 that is exposed from head body 50 in a state of being held by head body 50 and can clean a target object in an oral cavity (see FIGS. 9 and 17).

Specifically, head material 60 comprises: top wall part 61 having a substantially disk shape and placed on a placement surface (i.e., top surface 51a) of holder (i.e., base) 511; and flange 62 having a substantially cylindrical shape and extending from an outer peripheral edge of top wall part 61 toward a back side in front-back direction X (i.e., toward another side in the direction of central axis C of tubular part 52) See FIG. 10 that is a cross-sectional view of head material 60.

When holder 51 and head material 60 are inserted into tubular part 52 in a state where head material 60 is placed on holder 51, a central portion of top wall part 61 is exposed from first opening 521. That is, in the present exemplary embodiment, cleaning part 611 is formed in a portion of top wall part 61 exposed from first opening 521.

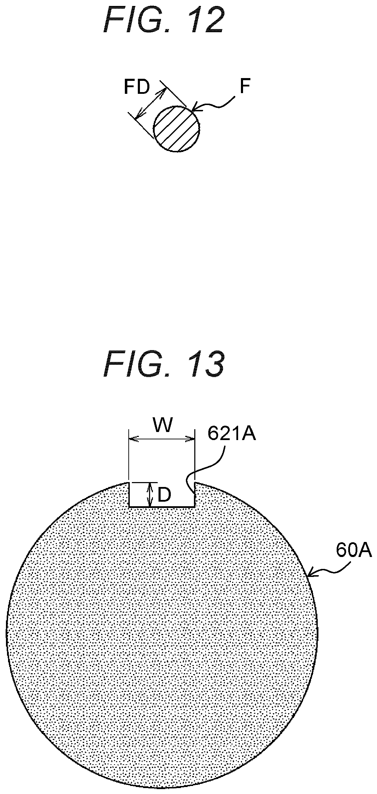

As illustrated in a plan view in FIG. 13, such head material 60 can be formed by applying heat to mold sheet-shaped member 60A made of a synthetic resin.

FIG. 11 is a view for explaining cleaning part 611 of head material 60. FIG. 12 is a cross-sectional view illustrating fiber F which is used in head material 60. Sheet-shaped member 60A as illustrated in FIG. 13 can be formed by applying an adhesive to an entire surface of cloth-shaped sheet portion F1, and bonding piles F2 formed by weaving fibers F, to the surface of sheet portion F1 (see FIG. 11).

Here, in the present exemplary embodiment, piles F2 are obtained by weaving fibers F such that a plurality of loops F2a having opening spaces S in the inside thereof are formed.

These piles F2 are bonded to the surface of sheet portion F1 such that the plurality of loops F2a are spread in a planar shape in a state where opening directions of opening spaces S are directed in substantially the same direction (in FIG. 11, a direction substantially perpendicular to the page).

A portion of head material 60 formed with piles F2 and exposed from first opening 521 serves as cleaning part 611.

In the present exemplary embodiment, the plurality of loops F2a of cleaning part 611 has height H ranging from 0.5 mm to 10 mm, inclusive. If height H of loops F2a is lower than 0.5 mm, performance of removing fouling such as tongue fur is deteriorated, and if height H of loops F2a is higher than 10 mm, loops F2a may be caught on a lingual papilla. In the present specification, the "performance of removing fouling such as tongue fur" may be referred to as "cleaning performance".

As illustrated in FIG. 12, fibers F forming piles F2 have fiber diameter FD ranging from 0.001 mm to 0.05 mm, inclusive. This is because, if fiber diameter FD of fibers F is smaller than 0.001 mm, the performance of removing fouling such as tongue fur is deteriorated, and if fiber diameter FD of fiber F is larger than 0.05 mm, touch on the tongue is deteriorated, and a sense of discomfort is generated at the time of use.

FIG. 13 is a plan view illustrating a sheet-shaped member usable for forming head material 60. Further, in the present exemplary embodiment, cutout 621A is formed in sheet-shaped member 60A as illustrated in FIG. 13, in order to cause the opening direction of opening spaces S of loops F2a to be a predetermined direction (i.e., so as not to make a mistake in direction) when head material 60 is held by head body 50. When sheet-shaped member 60A is molded, cutout 621 is formed in flange 62 alone (see FIG. 3).

Positioning projection 5122 to which cutout 621 of head material 60 is introduced is formed on an upper portion of tubular body 512 of holder (i.e., base) 51. Specifically, projection 5122 comprises prop wall 5122a extending upward, and arc-shaped wall 5122b connected to an upper end of prop wall 5122a (see FIGS. 14 and 15 that are perspective views of holder 51). By introducing (i.e., inserting) arc-shaped wall 5122b into cutout 621 of head material 60, head material 60 is held in a state of being positioned by holder (i.e., base) 51.

Here, in the present exemplary embodiment, cutout 621A is formed on one side in the same direction as the opening direction of opening space S in sheet-shaped member 60A. Thus, when head material 60 is held by head body 50, the opening direction of opening spaces S of loops F2a substantially corresponds to up-down direction Z. That is, in the present exemplary embodiment, by moving oral hygiene device nozzle 20 in up-down direction Z, fouling such as tongue fur can be scraped out by loops F2a.

As illustrated in FIGS. 3 and 17, in the present exemplary embodiment, through-hole 525 penetrating in up-down direction Z is formed at a lower end of tubular part 52 (i.e., at a portion connected to main body 31 of nozzle handle 30). This through-hole 525 has a lower end (i.e., one end) communicating with flow path P2 formed in nozzle handle 30, and an upper end (i.e., another end) communicating with liquid supply path P3. Then, liquid introduced from flow path P2 into through-hole 525 can be supplied to liquid supply path P3. As described above, in the present exemplary embodiment, through-hole 525 is a communication part of flow path P2 with liquid supply path P3.

Further, as illustrated in FIG. 7, in the present exemplary embodiment, a cross-sectional area (i.e., cross-sectional area taken along a horizontal plane) of through-hole 525 is made smaller than a cross-sectional area (i.e., area of a flow path cross section) of flow path P2, and this through-hole 525 is made to function as throttle part P2a formed in the communication part of flow path P2 with liquid supply path P3.

In this manner, when throttle part P2a is formed in the communication part of flow path P2 with liquid supply path P3, a flow rate (i.e., amount of liquid flowing per unit time) of the liquid supplied to liquid supply path P3 can be set to a predetermined amount, for example, by setting a cross-sectional area of throttle part P2a to a predetermined size. That is, a more suitable amount of liquid can be supplied to liquid supply path P3.

Further, in the present exemplary embodiment, as illustrated in FIG. 3, recess 5123 having a cutout shape is formed in a lower end part of tubular body 512 of holder 51. Therefore, the supply of the liquid from through-hole 525 to liquid supply path P3 is not disturbed by tubular body 512, when holder 51 is inserted into tubular part 52.

Here, in the present exemplary embodiment, a burden on the target object in the oral cavity can be further reduced when oral hygiene device nozzle 20 is used.

Specifically, liquid supply path P3 that communicates with flow path P2 and can supply liquid to fibrous cleaning part 611 is formed in head 40. Accordingly, when oral hygiene device nozzle 20 is used, the target object in the oral cavity can be scrubbed in a state where the liquid is held by fibrous cleaning part 611 (i.e., opening spaces S of loops F2a) with use of a surface tension of the liquid supplied from liquid supply path P3.

In the present exemplary embodiment, liquid supply path P3 comprises gap P3a formed between outer peripheral surface 512a of tubular body 512 and inner peripheral surface 52a of tubular part 52 (see FIG. 17). This gap P3a is formed near holder 51.

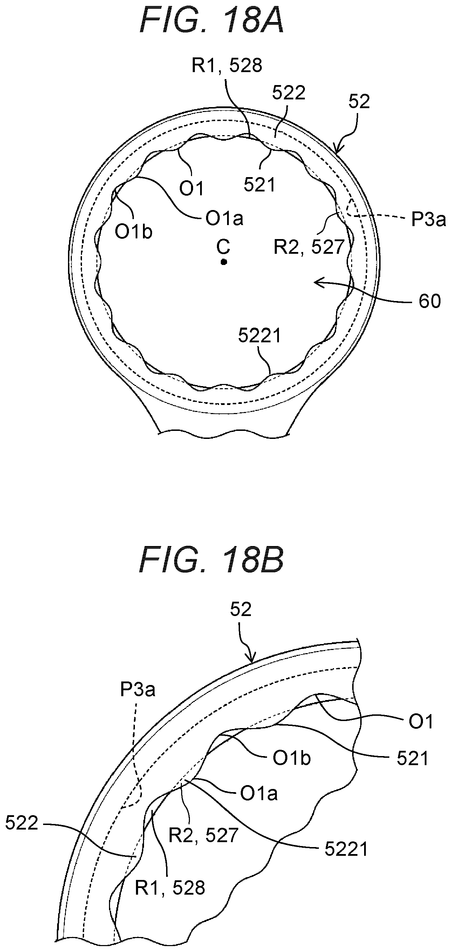

FIG. 18A is a view for explaining one example of retainer 527 and outlet 528 that have been formed in the head body. FIG. 18B is an enlarged view of a part of retainer 527 and outlet 528 in FIG. 18A. In the present exemplary embodiment, gap P3a is formed over the entire circumference on an outer peripheral side of holder 51 when a state where holder 51 is inserted into and held by tubular part 52 is viewed along the direction of central axis C of tubular part 52 (i.e., when viewed along front-back direction X). Therefore, gap P3a has an annular shape when head 40 is viewed from a front side (i.e., one side) in the direction of central axis C of tubular part 52 (see FIG. 18A).

As illustrated in FIG. 18B, at a peripheral edge of first opening 521 of tubular part 52, there are formed retainer 527 that suppresses come-off of head material 60 from head 40, and outlet 528 through which liquid in gap P3a is allowed to flow out toward the first opening 521.

In this manner, by forming retainer 527 and outlet 528 at the peripheral edge of first opening 521, the liquid can be spread to cleaning part 611 more reliably while come-off of head material 60 from head 40 (i.e., head body 50) is suppressed.

Specifically, when head 40 is viewed from the front side (i.e., one side) of central axis C of tubular part 52, covering region R2 covering holder (i.e., base) 51 from the front side is formed in tubular part 52, and this covering region R2 is made to function as retainer 527. Further, when head 40 is viewed from the front side (i.e., one side) of central axis C of tubular part 52, overlapping region R1 that overlaps with gap P3a is formed in first opening 521, and this overlapping region R1 is made to function as outlet 528.

Here, in the present exemplary embodiment, contour shape (i.e., a contour shape when head 40 is viewed from the front side in the direction of central axis C of tubular part 52) O1 of first opening 521 is made to be a curved waveform (hereinafter, which may be referred to simply as waveform) (see FIGS. 18A and 18B). In this way, when contour shape O1 of first opening 521 is a curved waveform, contour shape O1 of first opening 521 has at least one set of a protrusion and a recess. Contour shape O1 of first opening 521 is formed by making a contour shape of peripheral edge 522 of tubular part 52 into a curved waveform.

Then, retainer 527 is formed in portion O1a recessed toward the central axis C of contour shape O1 of first opening 521. This portion O1a recessed toward the central axis C is defined by portion 5221 protruding toward the central axis C in peripheral edge 522 of tubular part 52, and a distal end side of this portion 5221 protruding toward the central axis C is covering region R2 that covers holder (i.e., base) 51 from the front side. That is, the distal end side of portion 5221 protruding toward the central axis C serves as retainer 527 that suppresses come-off of head material 60 from head 40. In the present exemplary embodiment, claws 5221a to hook and hold head material 60 are formed in portions 5221 protruding toward the central axis C and located on both sides in up-down direction Z (see FIGS. 7 and 8).

On the other hand, portion O1b protruding to an outer peripheral side of contour shape O1 of first opening 521 serves outlet 528 through which liquid in gap P3a is allowed to flow out to the first opening 521.

Further, in the present exemplary embodiment, a plurality of sets of a protrusion and a recess have the same shape, and the plurality of sets of a protrusion and a recess are formed at substantially equal intervals over the entire circumference.

As described above, if contour shape O1 of first opening 521 is made to be a curved waveform, an edge is not formed in peripheral edge 522 of tubular part 52, and thus safety can be further enhanced when head 40 is inserted into the oral cavity and used.

Further, if a plurality of sets of a protrusion and a recess have the same shape, and the plurality of sets of a protrusion and a recess are formed at substantially equal intervals over the entire circumference, it becomes possible to cause cleaning part 611 to supply the liquid more evenly from the outer periphery.

Note that contour shape O1 of first opening 521 does not need to be a curved waveform, and can have various shapes.

### [Modification of configuration of oral hygiene device nozzle]

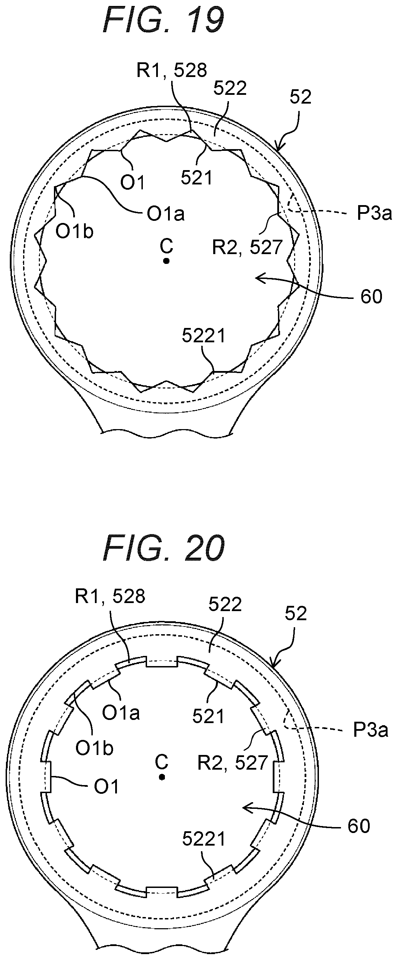

Next, a modification of a configuration of an oral hygiene device nozzle will be described with reference to FIGS. 19 to 22. FIGS. 19 to 22 are views illustrating first to fourth modifications, respectively, of retainer 527 and outlet 528 that are formed in head body 50.

First, as illustrated in FIG. 19, contour shape O1 of first opening 521 may be a triangular wave shape (i.e., a chevron shape in which straight lines have been connected).

In this way, also when contour shape O1 of first opening 521 is a triangular wave shape, contour shape O1 of first opening 521 has at least one set of a protrusion and a recess. This contour shape O1 of first opening 521 is formed by making a contour shape of peripheral edge 522 of tubular part 52 into a triangular wave shape.

Also in FIG. 19, gap P3a has an annular shape when head 40 is viewed from the front side (i.e., one side) in the direction of central axis C of tubular part 52.

Then, retainer 527 is formed in portion O1a recessed toward the central axis C of contour shape O1 of first opening 521, and outlet 528 is formed in portion O1b protruding toward the outer peripheral side of contour shape O1 of first opening 521.

Further, as illustrated in FIG. 20, contour shape O1 of first opening 521 may be a rectangular wave shape (that is, a protrusion and recess shape in which straight lines have been connected).

In this way, also when contour shape O1 of first opening 521 is a rectangular wave shape, contour shape O1 of first opening 521 has at least one set of a protrusion and a recess. This contour shape O1 of first opening 521 is formed by making a contour shape of peripheral edge 522 of tubular part 52 into a rectangular wave shape.

Also in FIG. 20, gap P3a has an annular shape when head 40 is viewed from the front side (i.e., one side) in the direction of central axis C of tubular part 52.

Retainer 527 is formed in portion O1a recessed toward the central axis C of contour shape O1 of first opening 521, and outlet 528 is formed in portion O1b protruding to the outer peripheral side of contour shape O1 of first opening 521.

Contour shape O1 of first opening 521 may be a sawtooth wave shape (i.e., a chevron shape in which straight lines have been connected).

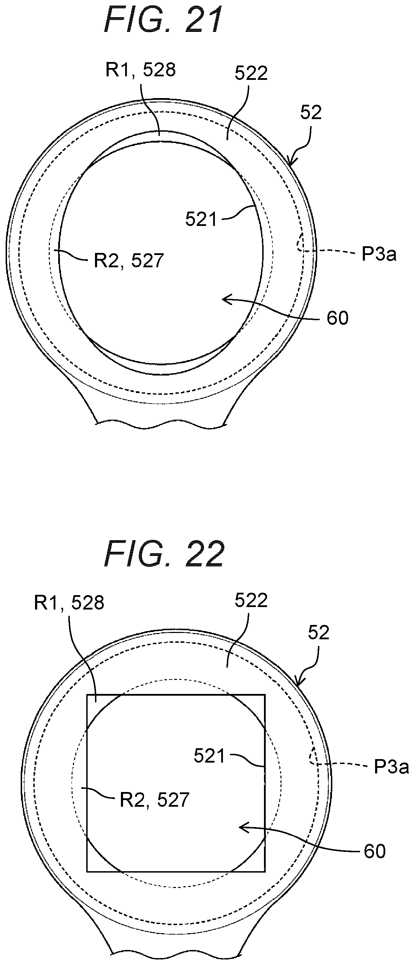

In addition, contour shape O1 of first opening 521 may be an elliptical shape as illustrated in FIG. 21. This contour shape O1 of first opening 521 is formed by making a contour shape of peripheral edge 522 of tubular part 52 into an elliptical shape.

Also in FIG. 21, gap P3a has an annular shape when head 40 is viewed from the front side (i.e., one side) in the direction of central axis C of tubular part 52.

Retainer 527 is formed on a short axis direction side of contour shape O1 of first opening 521, and outlet 528 is formed on a long axis direction side of contour shape O1 of first opening 521.

In addition, contour shape O1 of first opening 521 may be a quadrangular (i.e., polygonal) shape as illustrated in FIG. 22. This contour shape O1 of first opening 521 is formed by making a contour shape of peripheral edge 522 of tubular part 52 into a quadrangular (i.e., polygonal) shape.

Also in FIG. 22, gap P3a has an annular shape when head 40 is viewed from the front side (i.e., one side) in the direction of central axis C of tubular part 52.

Retainer 527 is formed at a central portion of a side of contour shape O1 of first opening 521, and outlet 528 is formed at an apex portion of contour shape O1 of first opening 521.

As described above, contour shape O1 of first opening 521 when head 40 is viewed from the front side (i.e., one side) in the direction of central axis C of tubular part 52 can be various shapes such as a waveform, a chevron shape, a protrusion and recess shape, an elliptical shape, and a quadrangular shape.

### [One example of method of assembling oral hygiene device nozzle]

Oral hygiene device nozzle 20 configured as described above can be assembled, for example, as follows.

First, head material 60 is held by holder (i.e., base) 51. At this time, by introducing (i.e., inserting) arc-shaped wall 5122b into cutout 621 of head material 60, head material 60 is held in a state of being positioned by holder (i.e., base) 51.

Then, holder (i.e., base) 51 in a state of holding head material 60 is inserted into tubular part 52 from the second opening 523 in a state where head material 60 faces forward in front-back direction X. Specifically, holder (i.e., base) 51 is inserted into tubular part 52 until peripheral edge 522 comes into contact with head material 60. Thus, head material 60 is sandwiched between holder (i.e., base) 51 and tubular part 52. Then, by locking portion to be locked 5121 of holder 51 to locking claw 526, holder 51 is held in a state where come-off of tubular part 52 inhibited.

At this time, holder (i.e., base) 51 is inserted into tubular part 52 in a state where arc-shaped wall 5122b of projection 5122 to be inserted into cutout 621 of head material 60 is positioned upward. In this way, through-hole 525 is exposed from recess 5123 having a cutout shape.

Next, cover 53 is inserted into tubular part 52 from the second opening 523 in a state where protrusion 532 faces forward in front-back direction X. Then, welding rib 5241 formed on peripheral edge 524 on the second opening 523 of tubular part 52 is welded to lid body 531 by ultrasonic welding or the like, to seal the cover 53 side (i.e., back side) of head 40.

Thus, oral hygiene device nozzle 20 is assembled. Note that the above assembling method is merely one example, and oral hygiene device nozzle 20 can be assembled by various methods.

### [One example of operation of oral hygiene device]

Next, an operation of oral hygiene device 1 to which oral hygiene device nozzle 20 described above has been attached will be described.

First, a user or the like holds grip 15 and inserts head 40 into an oral cavity to cause cleaning part 611 to face a target object in the oral cavity.

Next, while holding grip 15, the user or the like operates operation switch 15a to supply liquid.

Then, the target object in the oral cavity is scrubbed while the liquid is held by fibrous cleaning part 611 (i.e., opening spaces S of loops F2a). At this time, head material 60 is to be used while being washed with liquid. In this way, fouling in the oral cavity is removed.

Note that the method of using oral hygiene device nozzle 20 described above is merely one example, and oral hygiene device nozzle 20 can be used in various methods.

### [Operational effect]

Hereinafter, a characteristic configuration of the oral hygiene device nozzle (i.e., oral hygiene device member) described in the above exemplary embodiment and an effect obtained by the configuration will be described.

Oral hygiene device nozzle (i.e., oral hygiene device member) 20 described in the above exemplary embodiment comprises nozzle handle (i.e., handle) 30 having flow path P2 through which liquid can pass. Further, oral hygiene device nozzle 20 comprises head 40 having: head body 50 connected to one end side of nozzle handle 30; and head material 60 held by head body 50. Further, head material 60 comprises fibrous cleaning part 611 that is exposed from head body 50 in a state of being held by head body 50 and can clean a target object in an oral cavity. In head 40, liquid supply path P3 that communicates with flow path P2 and can supply liquid to cleaning part 611 is formed.

As a result, when a target object in an oral cavity such as a tongue is cleaned by using oral hygiene device nozzle 20, the target object can be cleaned by the cleaning part in a state where liquid is held. As a result, since direct contact of fibrous cleaning part 611 with the target object in the oral cavity such as the tongue is inhibited, a burden on the target object in the oral cavity can be further reduced.

In addition, since the target object in the oral cavity such as the tongue can be cleaned by cleaning part 611 while the liquid is supplied to the target object, the performance of removing fouling such as tongue fur can be further improved.

Since head material 60 can be used while being washed with the liquid, oral hygiene device nozzle 20 can be maintained in a cleaner state. Further, when head material 60 is used while being washed with the liquid, adhesion of fouling to head material 60 during use is suppressed, which also makes it possible to further improve the performance of removing fouling such as tongue fur.

As described above, according to the above exemplary embodiment, it is possible to provide oral hygiene device nozzle 20 capable of further reducing a burden on a target object in an oral cavity and capable of maintaining a cleaner state while suppressing deterioration in cleaning performance.

That is, oral hygiene device nozzle 20 described in the above exemplary embodiment has the above configuration, can keep head material 60 clean by using head material 60 while washing with the liquid, and has cleaning performance while reducing a burden on a target object in an oral cavity such as a tongue.

In addition, cleaning part 611 may be formed of piles.

This makes it possible to suppress deterioration in the performance of removing fouling such as tongue fur while further improving safety. In addition, by holding the liquid using the surface tension of the piles, it is possible to more reliably hold the liquid by cleaning part 611.

Further, cleaning part 611 may comprise a plurality of loops F2a. Furthermore, a height of loops F2a may range from 0.5 mm to 10 mm, inclusive.

This makes it possible to suppress deterioration in the performance of removing fouling such as tongue fur while even further improving safety. In addition, if cleaning part 611 comprises loops F2a, the liquid can be more reliably held by loops F2a.

Fiber diameter FD of each fiber F of cleaning part 611 may range from 0.001 mm to 0.05 mm, inclusive.

This makes it possible to suppress deterioration in the performance of removing fouling such as tongue fur while suppressing a sense of discomfort to be felt during use.

A thickness of head 40 may range from 5 mm to 15 mm, inclusive, and a maximum dimension of an outer diameter of head 40 may range from 15 mm to 40 mm, inclusive.

This suppresses a sense of discomfort to be felt when head 40 is put into the oral cavity, which makes it possible to suppress deterioration of a feeling of use of oral hygiene device nozzle 20.

Head body 50 comprises holder (i.e., base) 51 having top surface (i.e., placement surface) 511a on which head material 60 is placed. Then, liquid supply path P3 is formed near holder (i.e., base) 51.

This allows head material 60 to be firmly held by holder (i.e., base) 51, namely, held while deformation of head material 60 is suppressed, while allowing the liquid to be more reliably supplied to cleaning part 611.

Head body 50 comprises tubular part 52 that is connected to one end side of nozzle handle 30 and in which holder (i.e., base) 51 can be inserted and held. Then, in a state where holder (i.e., base) 51 has been inserted into and held by tubular part 52, gap P3a formed on an outer periphery of holder (i.e., base) 51 constitutes at least a part of liquid supply path P3.

This allows the liquid to be spread to cleaning part 611 with a simpler configuration.

Further, when a state where holder (i.e., base) 51 is inserted into and held by tubular part 52 is viewed along an axial direction of tubular part 52, gap P3a may be formed over the entire circumference on the outer peripheral side of holder (i.e., base) 51.

This allows the liquid to gradually permeate from the entire periphery of head material 60 toward a center of cleaning part 611. As a result, the liquid can be more reliably spread throughout the entire cleaning part 611.

Further, on one side in the axial direction of tubular part 52, there may be formed first opening 521 from which cleaning part 611 of head material 60 is exposed when holder (i.e., base) 51 is inserted into and held by tubular part 52 in a state where head material 60 is placed on top surface (i.e., placement surface) 511a. Further, at a peripheral edge of first opening 521, there may be formed retainer 527 that suppresses come-off of head material 60 from head 40 (i.e., head body 50), and outlet 528 through which liquid in gap P3a is allowed to flow out toward the first opening 521.

By forming retainer 527 that suppresses come-off of head material 60 from head 40 (i.e., head body 50) at the peripheral edge of first opening 521 as described above, disengagement of head material 60 from head 40 (i.e., head body 50) can be more reliably suppressed during use of oral hygiene device nozzle 20. Further, by forming outlet 528 through which the liquid in gap P3a is allowed to flow out toward the first opening 521 at the peripheral edge of first opening 521 from which cleaning part 611 is exposed, the liquid can be more reliably spread to cleaning part 611.

Tubular part 52 may have covering region R2 that covers holder (i.e., base) 51 from one side in the axial direction of tubular part 52 when head 40 is viewed from one side in the axial direction of tubular part 52. Further, first opening 521 may have overlapping region R1 that overlaps with gap P3a when head 40 is viewed from one side in the axial direction of tubular part 52. Covering region R2 may serve as retainer 527, and overlapping region R1 may serve as outlet 528.

In this way, if overlapping region R1 serves as outlet 528 while covering region R2 serves as retainer 527, retainer 527 and outlet 528 can be more easily provided.

Further, head body 50 may comprise cover 53 that closes second opening 523 on another side in the axial direction of tubular part 52.

This makes it possible to suppress leakage of the liquid in liquid supply path P3 from second opening 523, so that the liquid in liquid supply path P3 can be more efficiently supplied to cleaning part 611.

In addition, holder (i.e., base) 51 may comprise tubular body 512 extending from an outer periphery of top surface (i.e., placement surface) 511a to another side in the axial direction of tubular part 52. Cover 53 may comprise protrusion 532 that is inserted into tubular body 512 (i.e., inner space P3b) to reduce a volume of liquid supply path P3.

This can more reliably suppress remaining of liquid in liquid supply path P3 after oral hygiene device nozzle 20 is used. As a result, head body 50 can be maintained in a cleaner state.

In addition, throttle part P2a may be formed in the communication part of flow path P2 with liquid supply path P3.

In this manner, when throttle part P2a is formed in the communication part of flow path P2 with liquid supply path P3, a flow rate (i.e., amount of liquid flowing per unit time) of the liquid supplied to liquid supply path P3 can be set to a predetermined amount, for example, by setting a cross-sectional area of throttle part P2a to a predetermined size. That is, a more suitable amount of liquid can be supplied to liquid supply path P3.

### [Others]

Details of the oral hygiene device member according to the present disclosure have been described above. Since the above exemplary embodiment is for illustrating the technology in the present disclosure, various modifications, replacements, additions, omissions, or the like, can be made within the scope of the claims or equivalents thereof.

For example, the above exemplary embodiment shows an example in which liquid supply path P3 (i.e., gap P3a) is formed near holder (i.e., base) 51 (i.e., around an outer periphery of tubular body 512). However, the present disclosure is not limited to such a configuration, and liquid supply path P3 may be formed in holder (i.e., base) 51. Such a configuration can be obtained, for example, by providing a plurality of through-holes in top wall part 511 of holder (i.e., base) 51. This also makes it possible to more reliably supply liquid to cleaning part 611.

In addition, liquid supply path P3 may be formed near holder (i.e., base) 51 and in holder (i.e., base) 51.

Further, in the above exemplary embodiment, sheet portion F1 having water impermeability and having a surface coated with an adhesive has been exemplified as the sheet portion of head material 60, but a sheet portion having water permeability can also be used. Such a water-permeable sheet portion allows liquid to be supplied to cleaning part 611 from the entire back surface.

Additionally, in the above exemplary embodiment, piles F2 having loops F2a are exemplified, but hook-shaped piles can also be used.

In addition, specifications (e.g., a shape, a size, a layout, and the like) of head material 60, head 40, and other details can be changed as appropriate.

As described above, the oral hygiene device member according to the present disclosure can reduce a burden on a target object in an oral cavity, and can maintain a cleaner state while suppressing deterioration of cleaning performance. Therefore, the oral hygiene device member according to the present disclosure can be applied to a device capable of feeding liquid for washing an oral cavity, specifically, an oral cavity washing device or the like.

## Claims

1. An oral hygiene device member (20) comprising:
a handle (30) having a flow path (P2) through which liquid is allowed to pass; and
a head (40) comprising a head body (50) connected to one end side of the handle (30), and a head material (60) held by the head body (50),
wherein
the head material (60) comprises a cleaning part (611), the cleaning part (611) being exposed from the head body (50) in a state of being held by the head body (50), the cleaning part (611) is formed of fibers that clean a target object in an oral cavity, and
a liquid supply path (P3) is formed in the head (40), the liquid supply path (P3) communicating with the flow path (P2) and supplying liquid to the cleaning part (611) **characterized in that**
the head body (50) comprises a base (51) having a placement surface (511 a) on which the head material (60) is placed,
the head body (50) comprises a tubular part (52) that is connected to the one end side of the handle (30) and allows the base (51) to be inserted and held,
a through-hole (525) communicating with the flow path (P2) is formed at a portion of the tubular part (52) connected to the one end side of the handle (30),
in a state where the base (51) is inserted into and held by the tubular part (52), a gap (P3a) formed between an outer periphery of the base (51) and an inner peripheral surface of the tubular part (52) constitutes at least a part of the liquid supply path (P3), and
the liquid is supplied to the cleaning part (611) through the flow path (P2), the through-hole (525), and the gap (P3a).

2. The oral hygiene device member (20) according to claim 1, wherein the cleaning part (611) is formed of piles (F2).

3. The oral hygiene device member (20) according to claim 2, wherein
the cleaning part (611) comprises a plurality of loops (F2a), and
each of the loops (F2a) has a height ranging from 0.5 mm to 10 mm, inclusive.

4. The oral hygiene device member (20) according to any one of claims 1 to 3, wherein each of the fibers (F) has a fiber diameter ranging from 0.001 mm to 0.05 mm, inclusive.

5. The oral hygiene device member (20) according to any one of claims 1 to 4, wherein
the head (40) has a thickness (T) ranging from 5 mm to 15 mm, inclusive, and
a maximum dimension of an outer diameter (OD) ranging from 15 mm to 40 mm, inclusive.

6. The oral hygiene device member according to any one of claims 1 to 5,
wherein
the liquid supply path is formed in the base, wherein
an inner space (P3b) is formed in the base (51), and
the inner space (P3b) constitutes at least a part of the liquid supply path (P3).

7. The oral hygiene device member (20) according to any one of claims 1 to 6, wherein when a state where the base (51) is inserted into and held by the tubular part (52) is viewed along an axial direction of the tubular part (52), the gap (P3a) is disposed over an entire circumference on an outer peripheral side of the base (51).

8. The oral hygiene device member (20) according to any one of claims 1 to 7, wherein
a first opening (521) is formed at one side in an axial direction of the tubular part (52), the first opening (521) exposing the cleaning part (611) of the head material (60) in a state where the head material (60) has been placed on the placement surface (511a) and the base (51) has been inserted in and held by the tubular part (52), and
a retainer (527) and an outlet (528) are formed on a peripheral edge of the first opening (521), the retainer (527) suppressing come-off of the head material (60) from the head (40), the outlet (528) through which liquid in the gap (P3a) is allowed to flow out toward the first opening (521).

9. The oral hygiene device member (20) according to claim 8, wherein
the tubular part (52) comprises a covering region (R2) that covers the base (51) from the one side in the axial direction of the tubular part (52) when the head (40) is viewed from the one side in the axial direction of the tubular part (52), and the covering region (R2) serves as the retainer (527), and
the first opening (521) comprises an overlapping region (R1) that overlaps with the gap (P3a) when the head (40) is viewed from the one side in the axial direction of the tubular part (52), and the overlapping region (R1) serves as the outlet (528).

10. The oral hygiene device member (20) according to any one of claims 1 to 9, wherein
the head body (50) comprises a cover (53) that closes a second opening (523) on another side in the axial direction of the tubular part (52).

11. The oral hygiene device member (20) according to claim 10, wherein
the base (51) comprises a tubular body (512) extending from an outer periphery of the placement surface (511a) to the other side in the axial direction of the tubular part (512), and
the cover (53) comprises a protrusion (532) that is inserted into the tubular body (512) to reduce a volume of the liquid supply path (P3).

12. The oral hygiene device member (20) according to any one of claims 1 to 11, further comprising:
a throttle part (P2a) disposed in a communication part of the flow path (P2) with the liquid supply path (P3).

## Patentansprüche

1. Ein Mundhygienevorrichtungselement (20), umfassend:
einen Griff (30) mit einem Strömungspfad (P2), durch den Flüssigkeit strömen kann; und
einen Kopf (40), der einen Kopfkörper (50), der mit einer Endseite des Griffs (30) verbunden ist, und ein Kopfmaterial (60) umfasst, das von dem Kopfkörper (50) gehalten wird,
wobei
das Kopfmaterial (60) einen Reinigungsteil (611) umfasst, wobei der Reinigungsteil (611) von dem Kopfkörper (50) in einem Zustand, in dem er von dem Kopfkörper (50) gehalten wird, freigelegt ist, wobei der Reinigungsteil (611) aus Fasern gebildet ist, die ein Zielobjekt in einer Mundhöhle reinigen, und
ein Flüssigkeitszufuhrpfad (P3) in dem Kopf (40) ausgebildet ist, wobei der Flüssigkeitszufuhrpfad (P3) mit dem Strömungspfad (P2) in Verbindung steht und dem Reinigungsteil (611) Flüssigkeit zuführt,
**dadurch gekennzeichnet, dass**
der Kopfkörper (50) eine Basis (51) umfasst, die eine Platzierungsfläche (511a) aufweist, an der das Kopfmaterial (60) platziert ist,
der Kopfkörper (50) einen rohrförmigen Teil (52) umfasst, der mit der einen Endseite des Griffs (30) verbunden ist und es der Basis (51) erlaubt, eingesetzt und gehalten zu werden,
ein Durchgangsloch (525), das mit dem Strömungspfad (P2) in Verbindung steht, an einem Abschnitt des rohrförmigen Teils (52) ausgebildet ist, der mit der einen Endseite des Griffs (30) verbunden ist,
in einem Zustand, in dem die Basis (51) in den rohrförmigen Teil (52) eingesetzt und von diesem gehalten wird, ein Spalt (P3a), der zwischen einer Außenperipherie der Basis (51) und einer Innenperipheriefläche des rohrförmigen Teils (52) gebildeten ist, zumindest einen Teil des Flüssigkeitszufuhrpfads (P3) bildet, und die Flüssigkeit dem Reinigungsteil (611) durch den Strömungspfad (P2), das Durchgangsloch (525) und den Spalt (P3a) zugeführt wird.

2. Das Mundhygienevorrichtungselement (20) nach Anspruch 1, wobei der Reinigungsteil (611) aus Stapeln (F2) gebildet ist.

3. Das Mundhygienevorrichtungselement (20) nach Anspruch 2, wobei
der Reinigungsteil (611) eine Vielzahl von Schlaufen (F2a) umfasst, und
jede der Schlaufen (F2a) eine Höhe, die von 0,5 mm bis 10 mm, einschließlich, reicht, aufweist.

4. Das Mundhygienevorrichtungselement (20) nach irgendeinem der Ansprüche 1 bis 3, wobei jede der Fasern (F) einen Faserdurchmesser, der von 0,001 mm bis 0,05 mm, einschließlich, reicht, aufweist.

5. Das Mundhygienevorrichtungselement (20) nach irgendeinem der Ansprüche 1 bis 4, wobei
der Kopf (40) eine Dicke (T), die von 5 mm bis 15 mm, einschließlich, reicht, und eine maximale Abmessung eines Außendurchmessers (OD), die von 15 mm bis 40 mm, einschließlich, reicht, aufweist.

6. Das Mundhygienevorrichtungselement nach irgendeinem der Ansprüche 1 bis 5,
wobei
der Flüssigkeitszufuhrpfad in der Basis ausgebildet ist, wobei
ein Innenraum (P3b) in der Basis (51) ausgebildet ist, und
der Innenraum (P3b) zumindest einen Teil des Flüssigkeitszufuhrpfades (P3) bildet.

7. Das Mundhygienevorrichtungselement (20) nach irgendeinem der Ansprüche 1 bis 6, wobei, wenn ein Zustand, in dem die Basis (51) in den rohrförmigen Teil (52) eingesetzt und von diesem gehalten wird, entlang einer axialen Richtung des rohrförmigen Teils (52) gesehen wird, der Spalt (P3a) über einen gesamten Umfang auf einer Außenperipherieseite der Basis (51) angeordnet ist.

8. Das Mundhygienevorrichtungselement (20) nach irgendeinem der Ansprüche 1 bis 7, wobei
eine erste Öffnung (521) auf einer Seite in einer axialen Richtung des rohrförmigen Teils (52) ausgebildet ist, wobei die erste Öffnung (521) den Reinigungsteil (611) des Kopfmaterials (60) in einem Zustand freilegt, in dem das Kopfmaterial (60) auf die Platzierungsfläche (511a) platziert wurde und die Basis (51) in den rohrförmigen Teil (52) eingesetzt und von diesem gehalten wurde, und
ein Halter (527) und ein Auslass (528) an einem Peripherierand der ersten Öffnung (521) ausgebildet sind, wobei der Halter (527) das Ablösen des Kopfmaterials (60) vom Kopf (40) unterdrückt, wobei durch den Auslass (528) Flüssigkeit in dem Spalt (P3a) zu der ersten Öffnung (521) hin ausströmen kann.

9. Das Mundhygienevorrichtungselement (20) nach Anspruch 8, wobei
der rohrförmige Teil (52) einen Abdeckbereich (R2) umfasst, der die Basis (51) von der einen Seite in der axialen Richtung des rohrförmigen Teils (52) abdeckt, wenn der Kopf (40) von der einen Seite in der axialen Richtung des rohrförmigen Teils (52) gesehen wird, und der Abdeckbereich (R2) als der Halter (527) dient, und
die erste Öffnung (521) einen Überlappungsbereich (R1) umfasst, der sich mit dem Spalt (P3a) überlappt, wenn der Kopf (40) von der einen Seite in der axialen Richtung des rohrförmigen Teils (52) gesehen wird, und der Überlappungsbereich (R1) als der Auslass (528) dient.

10. Das Mundhygienevorrichtungselement (20) nach irgendeinem der Ansprüche 1 bis 9, wobei
der Kopfkörper (50) eine Abdeckung (53) umfasst, die eine zweite Öffnung (523) auf einer anderen Seite in der axialen Richtung des rohrförmigen Teils (52) verschließt.

11. Das Mundhygienevorrichtungselement (20) nach Anspruch 10, wobei
die Basis (51) einen rohrförmigen Körper (512) umfasst, der sich von einer Außenperipherie der Platzierungsfläche (511a) zur anderen Seite in der axialen Richtung des rohrförmigen Teils (512) erstreckt, und
die Abdeckung (53) einen Vorsprung (532) umfasst, der in den rohrförmigen Körper (512) eingesetzt ist, um ein Volumen des Flüssigkeitszufuhrpfades (P3) zu reduzieren.

12. Das Mundhygienevorrichtungselement (20) nach irgendeinem der Ansprüche 1 bis 11, weiterhin umfassend:
einen Drosselteil (P2a), der in einem Kommunikationsteil des Strömungspfads (P2) mit dem Flüssigkeitszuführpfad (P3) angeordnet ist.

## Revendications

1. Élément de dispositif d'hygiène buccale (20) comprenant :
une poignée (30) ayant un trajet d'écoulement (P2) à travers lequel du liquide est laissé à passer ; et
une tête (40) comprenant un corps de tête (50) raccordé à un côté d'extrémité de la poignée (30), et un matériau de tête (60) maintenu par le corps de tête (50),
le matériau de tête (60) comprenant une partie de nettoyage (611), la partie de nettoyage (611) étant exposée depuis le corps de tête (50) dans un état de maintien par le corps de tête (50), la partie de nettoyage (611) étant formée de fibres qui nettoient un objet cible dans une cavité buccale, et
un trajet d'alimentation de liquide (P3) étant formé dans la tête (40), le trajet d'alimentation de liquide (P3) communiquant avec le trajet d'écoulement (P2) et alimentant du liquide à la partie de nettoyage (611)
**caractérisé en ce que** le corps de tête (50) comprend une base (51) ayant une surface de mise en place (511a) sur laquelle le matériau de tête (60) est placé,
le corps de tête (50) comprenant une partie tubulaire (52) qui est raccordée à un côté d'extrémité de la poignée (30) et qui permet à la base (51) d'être insérée et maintenue,
un trou traversant (525) communiquant avec le trajet d'écoulement (P2) étant formé au niveau d'une portion de la partie tubulaire (52) raccordée au côté d'extrémité de la poignée (30), dans un état où la base (51) est insérée à l'intérieur et maintenue par la partie tubulaire (52), un espace (P3a) formé entre une périphérie externe de la base (51) et une surface périphérique interne de la partie tubulaire (52) constituant au moins une partie du trajet d'alimentation de liquide (P3), et
le liquide étant alimenté à la partie de nettoyage (611) à travers le trajet d'écoulement (P2), le trou traversant (525), et l'espace (P3a).

2. Élément de dispositif d'hygiène buccale (20) selon la revendication 1, la partie de nettoyage (611) étant formée de poils (F2).

3. Élément de dispositif d'hygiène buccale (20) selon la revendication 2,
la partie de nettoyage (611) comprenant une pluralité de boucles (F2a), et
chacune des boucles (F2a) ayant une hauteur s'étendant de 0,5 mm à 10 mm, inclus.

4. Élément de dispositif d'hygiène buccale (20) selon l'une quelconque des revendications 1 à 3, chacune des fibres (F) ayant un diamètre de fibre s'étendant de 0,001 mm à 0,05 mm, inclus.

5. Élément de dispositif d'hygiène buccale (20) selon l'une quelconque des revendications 1 à 4,
la tête (40) ayant une épaisseur (T) s'étendant de 5 mm à 15 mm, inclus, et une dimension maximale d'un diamètre externe (OD) s'étendant de 15 mm à 40 mm, inclus.

6. Élément de dispositif d'hygiène buccale selon l'une quelconque des revendications 1 à 5,
le trajet d'alimentation de liquide étant formé dans la base,
un espace interne (P3b) étant formé dans la base (51), et
l'espace interne (P3b) constituant au moins une partie du trajet d'alimentation de liquide (P3).

7. Élément de dispositif d'hygiène buccale (20) selon l'une quelconque des revendications 1 à 6, lorsqu'un état où la base (51) est insérée à l'intérieur et maintenue par la partie tubulaire (52) est visualisé le long d'un sens axial de la partie tubulaire (52), l'espace (P3a) étant disposé sur une circonférence entière sur un côté périphérique externe de la base (51).

8. Élément de dispositif d'hygiène buccale (20) selon l'une quelconque des revendications 1 à 7,
une première ouverture (521) étant formée sur un côté dans un sens axial de la partie tubulaire (52), la première ouverture (521) exposant la partie de nettoyage (611) du matériau de tête (60) dans un état où le matériau de tête (60) a été placé sur la surface de mise en place (511a) et la base (51) a été insérée à l'intérieur et maintenue par la partie tubulaire (52), et
un dispositif de retenue (527) et un orifice de sortie (528) étant formés sur un bord périphérique de la première ouverture (521), le dispositif de retenue (527) supprimant un détachement du matériau de tête (60) de la tête (40), l'orifice de sortie (528) à travers lequel du liquide dans l'espace (P3a) est laissé à s'écouler vers la première ouverture (521).

9. Élément de dispositif d'hygiène buccale (20) selon la revendication 8,
la partie tubulaire (52) comprenant une région de couverture (R2) qui recouvre la base (51) depuis le côté dans le sens axial de la partie tubulaire (52) lorsque la tête (40) est visualisée depuis le côté dans le sens axial de la partie tubulaire (52), et la région de couverture (R2) servant de dispositif de retenue (527), et
la première ouverture (521) comprenant une région chevauchante (R1) qui chevauche l'espace (P3a) lorsque la tête (40) est visualisée depuis le côté dans le sens axial de la partie tubulaire (52), et la région chevauchante (R1) servant d'orifice de sortie (528).

10. Élément de dispositif d'hygiène buccale (20) selon l'une quelconque des revendications 1 à 9,
le corps de tête (50) comprenant un couvercle (53) qui ferme une seconde ouverture (523) sur un autre côté dans le sens axial de la partie tubulaire (52).

11. Élément de dispositif d'hygiène buccale (20) selon la revendication 10,
la base (51) comprenant un corps tubulaire (512) s'étendant depuis une périphérie externe de la surface de mise en place (511a) vers l'autre côté dans le sens axial de la partie tubulaire (512), et
le couvercle (53) comprenant une protubérance (532) qui est insérée dans le corps tubulaire (512) pour réduire un volume du trajet d'alimentation de liquide (P3).

12. Élément de dispositif d'hygiène buccale (20) selon l'une quelconque des revendications 1 à 11, comprenant en outre :
une partie manette (P2a) disposée dans une partie de communication du trajet d'écoulement (P2) avec le trajet d'alimentation de liquide (P3).
